# EUROPEAN PATENT APPLICATION

(11) **EP 3 848 016 A1**
(43) Date of publication of application: **14.07.2021**
(21) Application number: 20150645.8
(22) Date of filing: 08.01.2020
(51) Int. Cl.: A61K 8/36, A61K 8/365, A61K 31/19, A61K 31/191, A61K 31/20, A61Q 11/00

(54) **ORAL CARE COMPOSITION CONTAINING FATTY ACID COMPONENTS FOR TREATING CARIES**

(71) Applicant: 3M Innovative Properties Company, Saint Paul, MN 55133-3427 (US)
(72) Inventor: Haeberlein, Ingo, 82362 Weilheim (DE); Schmid, Brenda, 85570 Markt Schwaben (DE); Hauke, Melanie, 82205 Gilching (DE); Safi, Ajmal, 85757 Karlsfeld (DE)
(74) Representative: Brem, Roland

(57) **Abstract**

The invention relates to an oral care composition comprising certain fatty acid components or salts thereof. The fatty acid components are useful in a method of treating caries by reducing the lactic acid release of lactic acid releasing bacteria in an oral biofilm in the mouth of a human being or animal.

The fatty acid component comprises a carboxyl moiety which is attached to a C₃ to C₇ moiety to which optionally one or more residues selected from methyl, methoxy or hydroxy can be attached, and/or wherein one alky moiety of the C₃ to C₇ moiety is replaced by a keto moiety.

The invention also relates to a kit of parts comprising Part A comprising the fatty acid or component, Part B comprising water and/or a carrier component, and optionally Part C comprising an application device.

## Description

### Field of the Invention

The invention relates to an oral care composition containing one or more fatty acid components or salts thereof. The oral care compositions and in particular the fatty acid components contained therein were found to be effective for treating caries or reducing the risk of getting caries lesions by reducing the lactic acid release of lactic acid producing bacteria in an oral biofilm in the mouth of a living human being or animal.

### Background

Dental plaque, which may include bacteria such as Streptococcus mutans, comprises a biofilm that forms on surfaces in the oral cavity. Dental plaque is at least partly responsible for dental caries, gingivitis, and periodontal diseases.

Bacteria in dental plaque metabolize carbohydrates (for example, simple sugars) in the mouth and produce organic acids that can demineralize tooth enamel, dentin, and cement. Dental plaque can serve as a substrate for the deposition of tartar or calculus. Build-up of dental plaque and calculus can lead to gingivitis and, ultimately, to periodontal disease.

A currently available method to remove dental plaque from teeth is mechanical removal with, for example, dental floss or a toothbrush. A toothbrush can aid in removing dental plaque from exposed surfaces of a tooth, and dental floss can aid in removing dental plaque from, for example, interproximal and subgingival surfaces. Proper and regular use of dental floss and a toothbrush can mechanically remove or reduce dental plaque, and can reduce the incidence of dental caries, gingivitis, and periodontal disease. Certain antimicrobial formulations are available (in the form of mouthwashes, rinses, and toothpastes, for example) to aid in the control and treatment of dental plaque, dental caries, gingivitis, and periodontal disease.

Prevention of caries often focuses on prevention of dental plaque formation and dental plaque removal from tooth surfaces.

Thus, various attempts have been made to provide an oral composition which is suitable for cleaning teeth and/or reducing the amount of plaque and oral biofilm.

Daily clinical experience, however, indicates that most of the dental plaque prevalent in patient's mouth do not necessarily cause tooth demineralization, irrespective of the dietary behaviour of patients. Thus, recent clinical publications indicate, that the amount of plaque, which is permanently present in patient's mouth, is almost little correlated with patient's caries risk.

Meanwhile, it is also reported that dental caries results from an imbalance of the metabolic activity in the individual dental biofilm. This reflects the daily clinical experiences that only a small fraction of dental plaque might end up in tooth demineralization. Even more, certain studies with dental biofilm revealed that the bacterial composition of dental plaque does not necessarily indicate the prevailing metabolic (caries) activity of the individual dental plaque.

Attempts to influence the formation of biofilm and/or prevent the formation of plaque are described in various documents.

US 2009/0202456 A1 (Prencipe et al.) suggests using salts of arginine in combination with certain acids for preparing oral care compositions.

### Description of the Invention

None of the attempts suggested so far is completely satisfying.

As noted above, proper and regular use of dental floss and a toothbrush can reduce the amount of dental plaque.

However, clinical experience seems to indicate that irrespective of daily use of dental floss and/or a toothbrush, dental biofilm remains present on many tooth surfaces.

Moreover, a biofilm matrix such as dental plaque may contribute to the isolation of bacteria from the protective effect of antimicrobial compounds and, thus, may interfere with the function of antimicrobial formulations such as mouthwashes, rinses, and toothpastes.

As a result, alternative methods and compositions to control or prevent the risk of caries are desirable.

In particular, it would be desirable to identify components which are able to influence the metabolic balance residing in oral biofilms in a patient's mouth.

Such components should be easy in administering and simple in use.

Ideally, the components should be easy to obtain or prepare and reasonable in price of manufacturing or purchase.

Further, the components should not have undesired side effects like bad taste or being astringent.

There is also a desire for a product which does not require the application of high fluoride dosages to a patient.

The components of the respective composition should in particular be active if used and/or applied in a permanent treatment.

One or more of the above objects are addressed by the invention described in the present text and claims.

In one embodiment the present invention features an oral care composition for treating caries or reducing the risk of getting caries, the oral care composition comprising a fatty acid component or salts thereof contained in a solvent and/or carrier, the fatty acid component comprising a carboxyl moiety which is attached to a C₃ to C₇ moiety to which optionally one or more residues selected from methyl, methoxy or hydroxy can be attached, and/or wherein one alky moiety of the C₃ to C₇ moiety is replaced by a keto moiety.

The invention is also related to a kit of parts comprising the oral care composition as described in the present text and claims and an application device.

Described is also a method of using the fatty acid component or the kit of parts as described in the present text and claims for producing an oral care composition.

A further aspect of the invention is directed to the use of the fatty acid component in a method for treating caries or reducing the risk of caries, the method comprising the step of bringing the fatty acid component in contact with an oral biofilm and/or hard dental tissue in the mouth of a living human being or animal as described in the present text and claims.

It has been found that the treatment of caries is particular effective for patients (human beings and animals) which have or suffer from xerostomia (sometimes also called "dry mouth disease").

Further, it was found the treatment is particular effective if the oral care composition is applied to marginal regions of hard dental tissue.

Examples of marginal regions of hard dental tissue are interproximal areas of teeth, e.g. the area between two neighbouring teeth, fissures being present on the surface of a tooth, marginal gap between a dental restoration (e.g. dental filling or dental crown) and the tooth substance.

Unless defined differently, for this description the following terms shall have the given meaning:
A "composition" is understood to be a mixture of two or more components.
A "dental or oral care composition" is a composition which is to be used in the dental field including the orthodontic area. In this respect the composition should be not detrimental to the patient's health and thus free of hazardous and toxic components being able to migrate out of the composition. Commercially available dental products have to fulfil certain requirements such as those given in DIN EN ISO 1942:2011-03.
As used herein, a "dental surface" or "hard dental tissue" refers to tooth structures (e.g., enamel, dentin, and cementum) and bone.
A "tooth structure" is any tooth structure, prepared or ready for preparation by the dentist. It can be a single tooth or two or more teeth. A tooth structure is also referred to as hard dental tissue in contrast to soft dental tissue (e.g. gingival).
"Caries" is understood as tooth decay, also known as dental caries which is a demineralization and/or breakdown of teeth due to acids produced by bacteria.
"Dental plaque" is understood as is a biofilm or mass of bacteria that grows on surfaces within the mouth.
The term "biofilm" refers to a matrix containing bacteria. A biofilm in an oral cavity can include bacteria, epithelial cells, leukocytes, macrophages and further oral exudate.
A "paste" is a substance that behaves as a solid until a sufficiently large load or stress is applied, at which point it flows like a fluid. Pastes typically consist of a suspension of granular material in a background fluid. The individual grains are jammed together like sand on a beach, forming a disordered, glassy or amorphous structure, and giving pastes their solid-like character. Pastes can be classified by their viscosity or their consistency comparable to dental impression material.
A "film forming component" is a substance which will cause a composition containing it to change from a liquid stage to a solid stage in such a manner as to form a film or coating on a surface. A definition of the term "film former" can also be found in DIN 55945 (1999-07-00). The term "film former" is often used in varnishes.
A "film" is a thin sheet or strip of a preferably flexible material. A coating of a surface with a material typically results in a film.
A "toothpaste" (dentifrice) is a cleaning agent for the daily individual care. It is typically used as a prophylactic measure against caries, gingivitis or periodontitis. In contrast to this, a "prophylaxis paste" is a product which is used by a profession such as a dentist or a dental hygienist to remove adherent deposits such as stain, plaque or tartar which may stick to the surface of a natural tooth, artificial tooth crown or bridge or filling material. A prophylaxis paste is therefore typically used on slowly rotating paste carrier (sometimes also referred to as prophy cups). Most of the commercially available prophylaxis pastes have a different viscosity compared to tooth pastes.
A "gel" is typically a colloidal system in which a porous matrix of interconnected particles spans the volume of a liquid medium. In general, gels are apparently solid, jelly-like materials. Both by weight and volume, gels are mostly liquid in composition and thus exhibit densities similar to liquids, however, have the structural coherence of a solid. An example of a common gel is edible gelatine. Many gels display thixotropy, that is, they become fluid when agitated, but re-solidify when resting.
A "solvent" means a liquid which is able to at least partially disperse or dissolve a component at ambient conditions (e.g. 23°C). A solvent typically has a viscosity below 5 or below 1 or below 0.1 Pa*s at 23°C.
A "particle" means a substance being a solid having a shape which can be geometrically determined. Particles can typically be analysed with respect to e.g. grain size.
The mean particle size of a powder can be obtained from the cumulative curve of the grain size distribution and is defined as the arithmetic average of the measured grain sizes of a certain powder mixture. Respective measurements can be done using commercially available granulometers (e.g. CILAS Laser Diffraction Particle Size Analysis Instrument).
"Keto residue or moiety" means C=O.
"Carboxyl residue or moiety" means COOH or COO⁻.
"Ambient conditions" mean the conditions which the inventive solution or composition is usually subjected to during storage and handling. Ambient conditions may, for example, be a pressure of 900 to 1100 mbar, a temperature of 10 to 40 °C and a relative humidity of 10 to 100 %. In the laboratory ambient conditions are adjusted to 20 to 25 °C and 1,000 to 1,025 mbar.
A composition is "essentially or substantially free of' a certain component, if the composition does not contain said component as an essential feature. Thus, said component is not wilfully added to the composition either as such or in combination with other components or ingredient of other components. A composition being essentially free of a certain component usually does not contain that component at all. However, sometimes the presence of a small amount of the said component is not avoidable e.g. due to impurities contained in the raw materials used.
As used herein, "a", "an", "the", "at least one" and "one or more" are used interchangeably. The terms "comprise" or "contain" and variations thereof do not have a limiting meaning where these terms appear in the description and claims. Also herein, the recitations of numerical ranges by endpoints include all numbers subsumed within that range (e.g., 1 to 5 includes 1, 1.5, 2, 2.75, 3, 3.80, 4, 5, etc.).
The terms "comprise" or "contain" and variations thereof do not have a limiting meaning where these terms appear in the description and claims. The term "comprise" shall include also the terms "consist essentially of' and "consist of'. "Consisting essentially of' means that specific further components can be present, namely those which do not materially affect the essential characteristic of the article or composition. "Consisting of' means that no further components should be present. The term "comprise" shall include also the terms "consist essentially of" and "consists of'.
"And/or" means one or both. E.g., the expression component A and/or component B refers to a component A alone, component B alone, or to both component A and component B.
Adding an "(s)" to a term means that the term should include the singular and plural form. E.g. the term "additive(s)" means one additive and more additives (e.g. 2, 3, 4, etc.).

Unless otherwise indicated, all numbers expressing quantities of ingredients, measurement of physical properties such as described below and so forth used in the specification and claims are to be understood as being modified in all instances by the term "about".

### Detailed Description

It has been found that by application of the fatty acid component or a composition containing the fatty acid component to an oral biofilm containing lactic acid releasing bacteria, the lactic-acid release of the lactic-acid releasing bacteria in the oral biofilm can be significantly reduced.

Depending on the context, the term "fatty acid component" also includes a composition containing the fatty acid component.

It was observed that by using the fatty acid component, the caries activity of the oral biofilm can significantly be lowered.

As the lactic-acid release capacity is typically correlated with the caries activity of the oral biofilm, the composition described in the present text provides an effective means for treating caries or reducing the risk of getting caries lesions.

Unexpectedly, this effect was observed even, if no further active components such as fluoride ions, hydroperoxides, hypochlorite, or heavy metal components etc. were present. This is in contrast to what is typically suggested in the prior art.

As outlined in the examples described below, an in-vitro human saliva derived microcosm biofilm was used to apply the fatty acid component described in the present text using different treating schemes.

In comparison to a control it was observed that even very low concentrations of the fatty acid component are effective in lowering the lactic acid release of bacteria being present in an oral biofilm in the presence of sucrose.

It was also found that the lowering of the lactic acid release is not or only little correlated to the formation of the biofilm mass.

Without wishing to be bound to a certain theory, it is assumed that the fatty acid component functions either as a biofilm metabolism control agent or as an agent for modifying the structure of the biofilm or as a kind of cell membrane modifying agent.

The cell membrane modifying agent primarily refers to the cell membrane of the cells of the bacteria forming the biofilm and of the extra-cellular membranes constituting the biofilm.

This may have an impact on the biofilm metabolic network, resulting in the lowering of lactic acid formation in presence of sugar such as sucrose.

Independent of the mechanism, the fatty acid component is effective for treating caries or reducing the risk of getting caries lesions.

Irrespective of the different side chains, all tested fatty acid components seem to be equivalently effective to lower the lactic acid formation in an oral biofilm.

The oral care composition described in the present text containing a fatty acid component as agent for treating caries or reducing the risk of getting caries is designed to move from a plaque formation inhibition composition or dental plaque removal composition or biofilm reducing composition to a composition allowing the control of the metabolic balance on dental plaque.

The invention shows that fatty acid components are effective to accomplish caries activity control in a dental biofilm, i.e. being able to influence the metabolic balance of the dental biofilm.

Further, compared to previously used active components, the fatty acid component proposed in the present text is easily available at reasonable costs and not harmful to the patient.

The fatty acid component contained in the oral care composition described in the present text is in particular provided for use in a method or therapy of treating caries or reducing the risk of caries by reducing the lactic acid release of lactic acid producing bacteria in an oral biofilm of a human or animal being.

The fatty acid component comprises a carboxyl moiety which is attached to a C₃ to C₇ moiety. The C₃ to C₇ moiety contains 3 to 7 alky subunits which are typically abbreviated as CHR^{x}.

Thus, the fatty acid component bears a carboxyl moiety and a C₃₋₇ alkyl group attached to it. These components are sometimes referred to short fatty acids.

The C₃ to C₇ moiety may contain one or more side groups or residues selected from methyl, methoxy or hydroxy.

Further, one alkyl subunit of the C₃ to C₇ moiety can be replaced by a keto moiety.

Thus, the fatty acid component described in the present text comprises the alpha, beta, gamma, and delta hydroxy or keto fatty acids, wherein one or more H atoms may optionally be substituted by methyl or methoxy groups.

Typically, the fatty acid component described in the present text does not bear more than 2 hydroxy moieties or 2 methyl moieties or 2 methoxy moieties or a combination thereof.

Comprised are also the orally and/or medically acceptable salts and hydrates thereof. Orally and/or medically acceptable salts include in particular the Li, Na, K and ammonium salts (such as NH₄⁺).

According to one embodiment the fatty acid component is characterized by the following formula, the respective salts and hydrates thereof:

H₂CR⁷-(CHR⁶)ᵣ-(CHR⁵)_{q}-(CHR⁴)ₚ-(CHR³)ₒ-(CHR²)ₙ-(CHR¹)ₘ-COOH

with
m+n+o+p+q+r being independently selected from 0 or 1,
m+n+o+p+q+r ≥ 2,
R¹⁻⁶ being independently selected from H, CH₃, OH and OCH₃,
R⁷ being independently selected from H, OH,
wherein one of CHR^{x} can be replaced by a keto moiety,
wherein not more than two of CHR^{x} can be replaced by a CH₃, OH or OCH₃ moiety,
with x being 1 to 6.

According to another embodiment the fatty acid component is characterized by the following formula, the respective salts and hydrates thereof:

H₂CR⁷-(CHR⁶)ᵣ-(CHR⁵)_{q}-(CHR⁴)ₚ-(CHR³)ₒ-(CHR²)ₙ-(CHR¹)ₘ-COOH

with
m+n+o+p+q+r being independently selected from 0 or 1,
m+n+o+p+q+r ≥ 2,
R¹ being independently selected from H and OH,
R² being independently selected from H, CH₃, and OH,
R³ being independently selected from H, CH₃, and OH,
R⁴ being independently selected from H, CH₃, and OH,
R⁵ being independently selected from H, CH₃, and OH,
R⁶ being independently selected from H, CH₃, and OH,
R⁷ being independently selected from H, OH,
wherein one of CHR^{x} can be replaced by a keto moiety,
wherein not more than two of CHR^{x} can be replaced by a CH₃, OH or OCH₃ moiety,
with x being 1 to 6.

In particular, the following fatty acid components were found to be useful: Butyric acid, 2-Ketobutyric acid, 2-Hydroxybutyric acid, Sodium 3-methyl-2-oxobutyrat, 2-Hydroxy-3-methylbutyric acid, 3-Methylbutryic acid, Pentanoic acid, 4-Oxopentanoic acid, 4-Methyl-2oxopentanoic acid, Hexanoic acid, 6-Hydroxyhexanoic acid, Heptanoic acid, Octanoic acid, 2-Hydroxy-octanoic acid, the respective salts and hydrates thereof, in particular the Li, Na, K or ammonium salts and hydrates thereof.

Mixtures of the fatty acid components can be used, if desired.

If desired, the fatty acid component can also be characterized by its molecular weight which is typically in the range of 100 to 180 g /mol.

Using a fatty acid component having a molecular weight in the above range was found to be beneficial because it allows an easier dissolution of the component in a solvent or carrier material. It may also help to facilitate the interaction of the fatty acid component with the cell membranes of the lactic acid reducing bacteria located in a biofilm.

The fatty acid component is typically present in the oral care composition in an amount being effective for reducing the lactic acid release of acetic acid releasing bacteria by at least 40 or at least 45 or at least 50% compared to a control composition not containing the fatty acid component.

The following amounts were found to be suitable:
Lower limit: at least 0.1 wt.% or at least 0.2 wt.%;
Upper limit: utmost 20 wt.% or utmost 10 wt.% or utmost 8 wt.%;
Range: 0.1 to 20 wt.% or 0.2 to 10 wt.% or 0.2 to 8 wt.%;
with respect to the weight of the whole composition.

According to one embodiment, the fatty acid component is used in combination with water. Thus, the oral care composition comprises one or more of the fatty acid components and water.

Water is used for dissolving or dispersing the fatty acid component, at least partially. Water is typically present in an amount sufficient to fully dissolve the fatty acid component and other optional components being present in the oral care composition.

Depending on the concentration of the fatty acid component used, the fatty acid component may be present in the form of micelles.

It has been found that the desired effect of reducing the lactic-acid release can be achieved more effectively, if the fatty acid component is present in dissolved form in a composition.

A fatty acid component containing oral care composition may comprise water in the following amounts:
Lower limit: at least 5 or at least 10 or at least 20 wt.%;
Upper limit: utmost 99.9 or utmost 95 or utmost 90 wt.%;
Range: 5 to 99.9 or 10 to 95 or 20 to 90 wt.%;
wt.% with respect to the whole fatty acid component containing oral care composition.

The following ratios were found to be effective for achieving the desired result.

The ratio of fatty acid component to water is typically at least 0.1 / 100, or at least 0.2 / 100 with respect to weight.

The ratio of fatty acid component to water can be in a range of 0.1 / 100 to 15 / 100, or in a range of 0.2 / 100 to 10 / 100 with respect to weight.

The fatty acid component may also be used in combination with a carrier component.

Thus, the oral care composition may comprise one or more fatty acid components, one or more carrier components and optionally water.

The nature and structure of the carrier component is not particularly limited, unless the desired result cannot be achieved.

Carrier components may help to adjust the rheological properties of the fatty acid component containing composition to support the application and/or to trigger the release of the active agent(s) contained in the composition.

If present, the carrier component is typically present in the following amount(s):
Lower limit: at least 1 or at least 2 or at least 5 wt.%;
Upper limit: utmost 90 or utmost 80 or utmost 70 wt.%;
Range: 1 to 90 or 2 to 80 or 5 to 70 wt.%;
wt.% with respect to the whole fatty acid component containing oral care composition.

Different kinds of carrier components can be used, if desired.

Suitable carrier components are gel-forming agents or paste-forming agents.

Useful examples of gel-forming or paste-forming agents include Irish moss, carboxymethyl cellulose, gum tragacanth, gum arabic, gum Karaya, sodium alginate, hydroxyethyl cellulose, methyl and ethyl cellulose, carrageenan, xanthan gum, polyvinyl pyrrolidone, and mixtures thereof.

These gel-forming or paste-forming agents do not have abrasive properties with respect to the tooth surface.

According to one embodiment, the carrier component is a film-forming agent.

Film-forming agents are suitable to improve adherence of the quaternary ammonium alkyl component(s) contained in the composition described in the present text to hard dental tissue, such as a tooth surface.

If film-forming agent(s) are present, the components contained in the composition described in the present text are typically dissolved or dispersed in the film forming component(s) and form together with the film forming component(s) a film or coating on the surface of hard dental tissue.

The molecular weight (Mw) of the film-forming agent can vary over a wide range (e.g. 2,000 to 1,200,000 g/mol). Typical ranges include 10,000 to 500,000 or 20,000 to 300,000 g/mol.

The molecular weight is typically provided by the manufacturer of the respective film-forming component. If desired, the molecular weight can be determined by GPC technology, using e.g. a polystyrene standard.

If the molecular weight of the film-forming agent is too low, the film forming component might not be able to form a sufficiently durable film or coating. Thus, the effect of a delayed release of the fatty acid component might not be obtained.

The film caused or produced by the film-forming agent typically has a thickness in a range of 0.5 µm to 100 µm or 10 µm to 50 µm.

Film formers or film-forming agents can be classified as natural film former, semi-synthetic film formers, cellulose derivatives, poly(meth)acrylates and vinyl polymers.

Examples of natural film-forming agents include shellac, mastix, sandarac, tolubalsam, dammar resin, benzoe resin, keratin, maizin, gum Arabic and gelatines.

Examples of semi-synthetic film-forming agents include gelatines treated with formaldehyde and salol (acetaldehyde phenol condensate).

Typical cellulose derivatives include cellulose acetate phthalate, hydroxyethylcellulose, hydroxypropylcellulose, methylcellulose, ethylcellulose and hydroxypropylmethyl cellulose phthalate.

Examples of poly(meth)acrylates include copolymers of (meth)acrylic esters and amino functional (meth)acrylates, copolymers of (meth)acrylic acid and methyl methacrylate, polyacrylamide, polyacrylic acid and salts thereof, in particular partial salts thereof, including sodium salts.

Examples of vinyl polymers include polyvinyl pyrrolidon, polyvinyl acetate phthalate (e.g. hydroxypropyl- and hydroxypropyl-methylcellulose), homo- and copolymers of polyvinylacetate, homo- and copolymers of polyvinylpropionate, styrene acrylics, ethylene vinyl acetate, poly-(hydroxyethyl methacrylate, poly(vinylethylene glycol acrylate, polyvinyl alcohol(s).

Particular examples for film-forming agent(s) include (e.g. fully or partially hydrolyzed) polyvinylalcohol, polymethylvinylether, polyvinylpyrrolidone, (e.g. aqueous) acrylic resin dispersions (e.g. Eudragit™, commercially available from Röhm), gelatine, polysaccharides (e.g. agarose), polyacrylamide, copolymers of vinylpyrrolidinone and acrylamide, hydrophilic cellulose derivatives (e.g. hydroxyethylcellulose, hydroxypropylcellulose, methylcellulose), homo- and copolymers of polyvinylacetate, homo- and copolymers of polyvinylpropionate, styrene acrylics, ethylene vinyl acetate, polyurethanes, hydroxylated acrylates such as poly(hydroxyethyl methacrylate), poly(vinylethylene glycol acrylate), and combinations and mixtures thereof.

Examples of film-forming agents which are sometimes preferred include polyvinyl alcohol, gelatine, polyacrylic acid, partially neutralized polyacrylic acid and mixtures thereof.

If a film-forming agent is present, it is typically present in the following amounts:
Lower limit: at least 5 or at least 10 or at least 20 wt.%;
Upper limit: utmost 90 or utmost 80 or utmost 70 wt.%;
Range: 5 to 90 or 10 to 80 or 20 to 70 wt.%;
wt.% with respect to the whole fatty acid containing oral care composition.

According to a further embodiment, the fatty acid component is used in combination with abrasive particles.

Thus, the oral care composition may comprise one or more fatty acid components, abrasive particles and optionally carrier component(s) and water.

Examples of abrasive particles which may be present include perlite, bentonite, silica, alumina, aluminium hydroxide, ilmenite (FeTiO₃), zircon oxide, zircon silicate, calcium carbonate, sodium bicarbonate, titanium dioxide, precipitated lime, chalk, flour of pumice, zeolites, talcum, kaolin, kieselguhr, aluminium oxide, silicates and mixtures thereof.

According to a further embodiment, the fatty acid component is used in combination with certain amino acids.

Thus, the oral care composition comprises one or more fatty acid components, one or more amino acids, water, optionally carrier component(s) and optionally abrasive particles.

There is no need for amino acids to be present, however, the presence of certain amino acids in combination with the fatty acid component may further help or even improve the efficacy or capacity of the oral care composition described in the present text.

The use of amino acids for reducing lactic acid release of lactic acid producing bacteria in an oral biofilm is described in European patent application number 18183601.6. The content of this application is herewith incorporated by reference. As described in EP 18183601.6, not all kinds of amino acids were found to be useful, but only a few.

By using a combination of the fatty acid component described in the present text in combination with certain amino acids has the following benefit.

On the one hand, the fatty acid component(s) may help to modifying the structure of the oral biofilm and thus have an influence on the overall environment where the lactic acid producing bacteria are located.

On the other hand, the amino acid(s) have a direct impact on the metabolism of the lactic acid releasing bacteria.

The following amino acids were found to be useful for achieving the desired results: glycine, leucine, isoleucine, lysine, methionine, phenylalanine, serine, threonine, valine, tryptophan and mixtures thereof, with the following amino acids being sometimes preferred: glycine, phenylalanine, serine, isoleucine, leucine, methionine, with glycine and phenylalanine being sometimes being even more preferred.

The amino acids may be natural or synthetic.

The amino acids might be in D- or L-configuration, wherein the L-configuration is preferred.

In contrast, the following amino acids were found to be not effective for reducing the lactic-acid release of bacteria in a biofilm: proline, arginine, histidine, aspartic acid, glutamine, tyrosine. These amino acids are therefore not suggested for this particular use.

According to one embodiment, the fatty acid component containing oral care composition described in the present text does typically not contain these amino acids in an effective amount, e.g. more than 0.5 or more than 0.3 or more than 0.1 wt.%.

On the other hand, the amino acids selected from glycine, leucine, isoleucine, lysine, methionine, phenylalanine, serine, threonine, valine, tryptophan and mixtures thereof may be present in an amount sufficient for achieving the desired result.

If present, the amino acids contained in the fatty acid component containing oral care composition are used in a therapeutically effective amount being sufficient to influence the lactic acid release metabolism of lactic acid releasing bacteria in an oral biofilm.

If present, the fatty acid component containing oral care composition and the amino acids contained therein are used for a time period being sufficient to influence the lactic acid release metabolism of lactic acid releasing bacteria in an oral biofilm.

More precisely, the fatty acid component containing oral care composition and the amino acids contained therein are typically present in an amount and applied for a time period effective to reduce the lactic acid release of lactic acid producing bacteria by at least 40% or at least 45% or at least 50% compared to the situation before the oral care composition described in the present text was used.

A suitable method for determining the effectiveness is described in the example section.

If present, the amino acid(s) are typically present in the following amounts:
Lower limit: at least 0.1 or at least 1 or at least 2 wt.%;
Upper limit: utmost 15 or utmost 12 or utmost 10 wt.%;
Range: 0.1 to 15 or 1 to 12 or 2 to 10 wt.%;
wt.% with respect to the whole fatty acid component containing oral care composition.

Depending on the amino acid used, certain concentrations might be even more effective than others.

If present, the following amounts were found to be particular effective:
glycine in an amount of 0.1 to 10 wt.%; or
leucine in an amount of 0.1 to 5 wt.%; or
isoleucine in an amount of 0.1 to 5 wt.%; or
lysine in an amount of 0.1 to 10 wt.%; or
methionine in an amount of 0.1 to 10 wt.%; or
phenylalanine in an amount of 0.1 to 5 wt.%; or
serine in an amount of 0.1 to 10 wt.%; or
threonine in an amount of 0.1 to10 wt.%; or
valine in an amount of 0.1 to 8 wt.%; or
tryptophan in an amount of 0.1 to 2 wt.%;
wt.% with respect to the whole fatty acid component containing oral care composition.

If present, the following concentrations or amounts were found to be effective, as well:
glycine in an amount of 1 to 10 wt.%; or
leucine in an amount of 1 to 5 wt.%; or
isoleucine in an amount of 2 to 5 wt.%; or
lysine in an amount of 5 to 10 wt.%; or
methionine in an amount of 2 to 10 wt.%; or
phenylalanine in an amount of 1 to 5 wt.%; or
serine in an amount of 1 to 10 wt.%; or
threonine in an amount of 6 to10 wt.%; or
valine in an amount of 3 to 8 wt.%; or
tryptophan in an amount of 0.5 to 2 wt.%;
wt.% with respect to the whole fatty acid component containing oral care composition.

The following ratios were found to be particularly effective for achieving the desired result.

If present, the ratio of amino acid(s) to water is typically at least 0.1 / 100, or at least 1.5 / 100, or at least 2 / 100 with respect to weight.

If present, the ratio of amino acid(s) to water can be in a range of 0.1 / 100 to 15 / 100, or in a range / 1.5 to 100 to 12 / 100, or in a range of 2 to 100 to 10 to 100 with respect to weight.

If amino acid(s) are present, the ratio of fatty acid component to amino acid(s) is typically at least 1 / 100 or at least 1 / 10 or at least 1 / 5 or at least 1 / 1 with respect to weight.

If present, the ratio of fatty acid component to amino acid(s) can be in a range of in a range 1 / 100 to 100 / 1, or in a range of 1 / 10 to 10 / 1 or in a range of 1 / 5 to 5 / 1 or in a range of 1 / 2 to 2 / 1 with respect to weight.

The fatty acid component containing oral care composition described in the present text may also comprise additive(s). One or more additives can be present, if desired.

If present, additive(s) are typically present in the following amount(s):
Lower limit: at least 0.01 or at least 0.1 or at least 0.5 wt.%;
Upper limit: utmost 10 or utmost 8 or utmost 6 wt.%;
Range: 0.001 to 10 or 0.1 to 8 or 0.5 to 6 wt.%;
wt.% with respect to the whole fatty acid component containing oral care composition.

Additive(s) which might be present include stabilizer(s), colourant(s), phosphate releasing agent(s), calcium releasing agent(s), anti-microbial agent(s), buffer(s), humectant(s), preservative agent(s), flavour additive(s) and mixtures thereof.

The composition might contain one or more stabilizer(s) as an additive.

If a stabilizer is present, the storage stability of the composition might be improved. That is, the individual components of the composition do not separate over time. A composition is defined as storage-stable, if the components do not separate from each other within 6 months or 12 months or 24 months or 36 months at ambient conditions.

If a stabilizer is present, it is typically, present in a low amount.

Amounts, found to be useful, include 0.01 to 3 wt.% or 0.1 to 1 wt.% with respect to the weight of the whole composition.

Examples of stabilizer(s) include copolymers of 2,5-furandione with 1,9-decadiene and methoxy-ethene (e.g. Stabilize™, International Specialty Products (ISP) Comp.) and carboxy vinyl polymers (e.g. Carbopol™, Lubrizol Advanced Materials Comp.).

Stabilizers typically have a mean particle size below 500 µm or below 250 µm or below 100 µm.

In a further embodiment, the composition comprises one or more colourants.

However, there is no need for a colourant to be present at all.

If a colourant is present, it is typically present in an amount of at most 5 wt.% or of at most 3 wt.% or of at most 1 wt.% with respect to the whole composition. Typical ranges include 0.01 wt.% to 5 wt.% or 0.1 wt.% to 3 wt.% with respect to the whole composition.

The presence of a colourant may allow an easy detection in a patient's mouth (especially compared to oral tissue and/or tooth substance) and control whether after the treatment all residues of the composition have been removed. E.g., a blue, green or violet colour may be suitable. Colouring of the dental composition can be achieved by incorporating colorants or pigments (organic and inorganic) into the composition.

Examples of colourants include red iron oxide 3395, Bayferrox™ 920 Z Yellow, Neazopon™ Blue 807 (copper phthalocyanine-based dye) or Helio Fast Yellow ER and mixtures thereof.

In a further embodiment, the composition comprises one or more phosphate releasing agent(s) as an additive.

However, there is no need for a phosphate releasing agent to be present at all.

If a phosphate releasing agent is present, it is typically present in an amount of at most 5 wt.% or of at most 3 wt.% or of at most 2 wt.% with respect to the whole composition. Typical ranges include 0.01 wt.% to 5 wt.% or 0.1 wt.% to 3 wt.% with respect to the whole composition.

Examples of phosphate and/or calcium releasing agent(s) include calcium pyrophosphate, calcium carbonate, dicalcium phosphate dehydrate, amorphous calcium phosphate, casein phosphopeptide, calcium sodium phosphosilicate, trimetaphosphate, and mixtures thereof.

In another embodiment, the composition comprises anti-microbial agent(s).

However, there is no need for an anti-microbial agent to be present at all.

If an anti-microbial agent it is present, it is typically present in an amount of at most 2 wt.% or of at most 1 wt.% or of at most 0.5 wt.% with respect to the whole composition. Typical ranges include 0.01 wt.% to 2 wt.% or 0.1 wt.% to 1 wt.% with respect to the whole composition.

The presence of an anti-microbial agent might help reducing health risks for professionals in the dental offices and laboratories as well as for patients.

Useful anti-microbial agents include chlorhexidine or derivatives thereof and aldehydes (glutaraldyde, phthalaldehyde) and chlorhexidine or its derivatives and salts of phenolics or acids. It can also be preferred to use acid adducts of chlorhexidine or its derivatives like e.g., acetates, gluconates, chlorides, nitrates, sulphates or carbonates.

Chlorhexidine and its derivatives (hereinafter referred to as CHX) are commercially available in water-based solutions (e.g. a 20 % aqueous solution of CHX digluconate, CAS 18472-51-0) or as a pure compound or as a salt. As additive the pure Chlorohexidine compound (CAS 55-56-1) and CHX salts like CHX diacetate monohydrate (CAS 56-95-1) or CHX dihydrochloride (CAS 3697-42-5) are preferred.

CHX also seems to be especially suited as an additive due in part to its well-known and proven anti-microbial action against Gram-positive and Gram-negative microorganisms including the oral Streptococci and Lactobacilli. CHX is bacteriostatic for Mycobaterium. CHX is also active against yeasts including Candida albicans and viruses including HIV, HBV, HCV, Influenza- and Herpes virus. A further advantage of CHX is its low toxicity.

Preferred anti-microbial agents include hexitidin, cetypyridiniumcloride (CPC), chlorhexidin (CHX), triclosan, stannous chloride, benzalkonium chloride, non-ionic or ionic surfactants (e.g. quaternary ammonium compounds), alcohols [monomeric, polymeric, mono-alcohols, polyalcohols (e. g. xylitol, sorbitol), aromatic (e. g. phenol)], antimicrobial peptides (e. g. histatins), bactericins (e. g. nisin), antibiotics (e. g. tetracycline), aldehydes (e. g. glutaraldehyde) inorganic and organic acids (e. g. benzoic acid, salicylic acid, fatty acids) or there salts, derivative of such acids such as esters (e. g. p-hydroxy benzoate or other parabenes, laurizcidin), enzymes (e. g. lysozyme, oxidases), proteins (e. g. enamel matrix protein, proline rich proteins), fluoride, EDTA, essential oils (e. g. thymol).

In another embodiment, the composition can comprise one or more buffer(s) as an additive.

However, there is no need for a buffer to be present at all.

If a buffer is present, it is typically present in an amount of at most 5 wt.% or of at most 3 wt.% or of at most 2 wt.% with respect to the whole composition. Typical ranges include 0.1 wt.% to 5 wt.% or 1 wt.% to 3 wt.% with respect to the whole composition.

Examples of buffers, which can be used, include acetic acid/acetate, tris(hydroxymethyl)aminomethane (TRIS), N-(2-acetamido)-2-aminoethane sulfonic acid (ACES), N-(2-acetamido)immino-diacetate (ADA), N,N-bis(2-hydroxyethyl)-2-aminoethane sulfonic acid (BES), 2,2-bis-(hydroxyethyl)-iminotris(hydroxylmethyl)methane (BIS-TRIS), 2-(cyclohexylamino)ethane sulfonic acid (CHES), 2-[4-(2-hydroxyethyl-1-piperazine)]ethane sulfonic acid (HEPES), 3-[4-(2-hydroxyethyl-1-piperazinyl)]propane sulfonic acid (HEPPS), 2-morpholinoethane sulfonic acid (MES), 3-morpholinopropane sulfonic acid (MOPS), piperazine-1,4-bis(2-ethane sulfonic acid (PIPES), N-[tris(hydroxymethyl)-methyl]-2-aminoethane sulfonic acid (TES), N-[tris(hydroxymethyl)-methyl]-glycine (TRICINE), and phosphate buffers, in particular hydrogen phosphate / dihydrogen phosphate buffers such as Na₂HPO₄, NaH₂PO₄, K₂HPO₄, KH₂PO₄.

The composition might contain one or more humectant(s) as an additive.

However, there is no need for a humectant to be present at all.

If a humectant is present, it is typically present in an amount of at most 5 wt.% or of at most 3 wt.% or of at most 2 wt.% with respect to the whole composition. Typical ranges include 0.01 wt.% to 5 wt.% or 0.1 wt.% to 3 wt.% with respect to the whole composition.

Examples of humectant(s) which can be used include glycerine, sorbitol, mannitol, xylitol, propylene glycol, polyethylene glycol and mixtures thereof.

Examples of preservative agent(s) which can be present include sodium benzoate, citric acid and its salts, and combinations thereof.

Examples of flavour additive(s) which can be used include peppermint, spearmint, and combinations thereof.

According to one embodiment, a fatty acid component containing oral care composition comprises or essentially consists of or consists of:
Component A (fatty acid) in an amount of 0.1 to 10 wt.%;
Component B (water) in an amount of 5 to 98 wt.%;
Component C (carrier component) in an amount of 0 to 90 wt.%;
Component D (amino acid) in an amount of 0 to 15 wt.%;
Component E (additive) in an amount of 0 to 10 wt.%;
wt.% with respect to the whole composition.

According to one embodiment, a fatty acid containing oral care composition comprises or essentially consists of or consists of:
Component A (fatty acid) in an amount of 0.1 to 10 wt.%;
Component B (water) in an amount of 5 to 98 wt.%;
Component C (carrier component) in an amount of 1 to 90 wt.%;
Component D (amino acid) in an amount of 0 to 15 wt.%;
Component E (additive) in an amount of 0 to 10 wt.%;
wt.% with respect to the whole composition.

According to one embodiment, a fatty acid containing oral care composition comprises or essentially consists of or consists of:
Component A (fatty acid) in an amount of 0.1 to 10 wt.%;
Component B (water) in an amount of 5 to 98 wt.%;
Component C (carrier component) in an amount of 1 to 90 wt.%;
Component D (amino acid) in an amount of 0.1 to 15 wt.%;
Component E (additive) in an amount of 0 to 10 wt.%;
wt.% with respect to the whole composition.

Using buffers may help to adjust the pH value of the composition. The composition is typically adjusted to a pH value in the range of 6 to 8. Thus, the composition is essentially neutral.

The composition can also be characterized by its viscosity. Depending on its chemical formulation, the viscosity may vary over a huge range.

If the composition is provided as liquid, the viscosity is typically in a range of 1 to 10 mPa*s or 1 to 1,000 mPa*s at 23°C.

If the composition is provided as gel or paste, the viscosity is typically in a range of 2,000 to 200,000 mPa*s at 23°C.

If desired, the viscosity of liquids can be measured using a Physica MCR 301 Rheometer (Anton Paar, Graz, Austria) with a cone/plate geometry CP25-1 under controlled shear rate at 23 °C (e.g. 100 s⁻¹). The diameter is 25 mm, the cone angle 1°, and the separation between the cone tip and the plate 49 µm.

If desired, the viscosity of pastes can be determined using a Physica MCR 301 Rheometer (Anton Paar, Graz, Austria) with a plate/plate geometry (PP15) at a constant shear rate of 1 s⁻¹ in rotation at 28 °C. The diameter of the plates is 10 mm and the gap between the plates is set to 2.0 mm.

The fatty acid component described in the present text is particularly useful for producing an oral care composition.

The oral care composition containing the fatty acid component described in the present text can typically be produced as follows:
The fatty acid component is provided and mixed with the other components of the oral care composition such as water, the carrier component(s), amino acid(s) and the additive(s), if present.

Depending on the nature of the optional carrier component(s) and additive(s), the mixing is done by dissolving or dispersing the fatty acid component in the carrier component(s) and additive(s), if desired, with the aid of a mixing device.

The mixing can also be accomplished shortly before the use of the oral care composition or the oral care composition can already be provided in a mixed and storage stable form.

The present invention is also directed to a kit of parts for use in a method or therapy of treating or reducing the risk of getting caries.

The kit of parts typically comprises
Part A comprising the fatty acid component described in the present text,
Part B comprising water for dissolving or dispersing the fatty acid component, optionally in combination with a carrier component,
and optionally Part C comprising an application device.

The other components, such as amino acid(s), carrier component(s) or additive(s) as described in the present text can be present in Part A or Part B or Part A and Part B, as desired.

Providing the components of the composition in separated parts can be beneficial to improve the storage stability.

Before use, the practitioner will prepare the oral care composition by combining the respective components of the individual parts.

The application of the oral care composition can be done by various means and/or using various devices.

Possible application devices include cups, sponges, brushes, dental trays, syringes, mouth guards, and clear tray aligners.

Clear tray aligners can straighten a dental patient's teeth without the need for using wires and brackets of traditional braces. The aligners typically consist of a sequence of clear, removable trays that fit over the teeth to straighten them.

Using the composition or kit of parts described in the present text in combination with a dental tray, mouth guard or clear tray aligner can be advantageous, as these kinds of devices are typically worn for a longer period of time (e.g. 10 min to 12 hrs) and thus are well suited for applying the oral care composition described in the present text for a longer period of time, if desired.

For use of the fatty acid component containing oral care composition described in the present text in combination with a dental tray, mouth guard or clear tray aligner, providing the fatty acid composition in the form of a paste or gel was found to be advantageous.

During storage, the oral care composition described in the present text is typically packaged in a suitable packaging device.

The size and shape of the packaging device typically depends on the form how the composition is provided.

Suitable packaging devices include sealable bottles, tubes, vessels or foil bags (including glass or plastic bottles, e.g. equipped with a screw cap), blisters, syringes, etc.

The packaging device might be designed for single-use or repeated use.

The fatty acid component can be used and applied in different forms or shapes.

According to one embodiment, the fatty acid component is provided in a liquid, e.g. in the form of an oral rinse or a mouth wash.

According to another embodiment, the composition is provided as part of a gel.

According to another embodiment, the composition is provided as part of a paste, e.g. in the form of a tooth paste.

According to another embodiment, the composition is provided as part of a gum, e.g. in the form of a chewing gum.

According to another embodiment, the composition is provided in a form which results in a coating after application. Such an application typically contains a film forming agent.

Other preventive care products where the fatty acid component described in the present text can be used include prophy pastes, prophy powder, varnishes, coatings, etc.

For causing the desired effect, the fatty acid component has to be brought in contact with the oral biofilm or hard dental tissue. The oral biofilm is typically located on tooth surfaces, in particular hard dental tissue.

The bringing into contact can be achieved by different means, including rinsing, spraying, brushing, swabbing, coating or combinations thereof.

The bringing into contact is typically done for a time period being sufficient for causing the desired effect.

The bringing into contact is typically done for a duration of at least 1 min or of at least 2 min or at least 3 min or at least 4 min.

If desired, the step of bringing into contact can be repeated several times.

According to one embodiment, the oral care composition is applied in periodic application scheme.

Possible daily repeating schemes for a periodic application scheme are:
at least 2-times for 1 to 5 min within 24 hours;
at least 3-times for 1 to 5 min within 24 hours.

These kinds of repeating schemes are typically applied, if the fatty acid component is provided in the form of a mouth wash or tooth paste.

According to one embodiment, the oral care composition is applied in a continuous application scheme.

Possible daily repeating schemes for a continuous application scheme are:
at least 1 hour within 24 hours;
at least 5 hours within 24 hours.

These kinds of repeating schemes are typically applied, if the fatty acid component is provided in the form of a gel or varnish to be applied either directly on the surface of the tooth structure or with the help of an application device.

Both application schemes can be repeated, if desired.

According to a further embodiment, the fatty acid component described in the present text is applied to form a coating or film which can persist on a tooth surface for at least 1, 2, 3 or 5 or 10 days up to 12 months or even longer.

In order to form a coating or film, the fatty acid component is applied together with film-forming agent(s).

Suitable film-forming agents are described above.

According to certain embodiments, the fatty acid component containing oral care composition described in the present text does typically not comprise the following components alone or in combination:
oxidizing components in an amount of more than 0.5 wt.%;
heavy metal components comprising Zn, Sn or Cu in an amount of more than 0.1 wt.%;
fluoride anions in an amount of more than 0.2 or more than 0.15 or more than 0.1 wt.%; wt.% with respect to the whole composition.

Certain embodiments of the composition described in the present text are essentially free of abrasive particles, in particular free of the abrasive particles described in the text above.

Essentially free means less than 1 or less than 0.5 or less than 0.1 wt.% or do not contain abrasive particles at all. Common to most of these substances is typically a comparable high hardness, e.g. above about Mohs 4 or above about 5.

Certain embodiments of the composition described in the present text are essentially free of oxidizing component(s) (e.g. less than 0.5 or less than 0.3 or less than 0.1 wt.%) or do not contain oxidizing components at all.

Oxidizing component(s) which are typically not present are peroxide, hypochlorite, perborate, persulfate, peroxyphosphate, peroxycarbonate.

Sometimes oxidizing components have a negative effect in that they may react with the amino acids being present in the composition having the result that the reaction products are no longer suitable for acting as a metabolism modifying agent.

Certain embodiments of the composition described in the present text are essentially free of heavy metal component(s), in particular those comprising Zn, Sn or Cu (e.g. less than 0.1 or less than 0.05 or less than 0.01 wt.%) or do not contain heavy metal component(s) at all.

Similarly, sometimes heavy metal components may have a negative effect, too, in that they may react with the amino acids being present in the composition (e.g. by forming insoluble complexes) having the result that the reaction products are no longer suitable for acting as a metabolism modifying agent.

However, unavoidable traces of either of these components in the raw materials used for producing the composition may nevertheless be present.

All components used in the composition described in the present text should be sufficiently biocompatible, that is, the composition should not produce a toxic, injurious, or immunological response in living tissue.

Further embodiments of the invention are given below:

### Embodiment 1

The fatty acid component described in the present text for use in a method of treating caries or reducing the risk of getting caries by reducing the lactic acid release of lactic acid producing bacteria in an oral biofilm in the mouth of a living human or animal,
the fatty acid component being applied in combination with water,
the fatty acid component being contained in an amount of at least 0.1 wt.% with respect to
the weight of the whole composition,
the composition having a pH in the range of 6 to 8 and
having a viscosity of 1 to 1,000 mPa*s at 23°C.

### Embodiment 2

The fatty acid as described in the present text for use in a method of treating caries or reducing the risk of getting caries by reducing the lactic acid release of lactic acid producing bacteria in an oral biofilm in the mouth of a living human or animal,
the fatty acid component being applied in combination with water and gel-forming or paste-forming component(s),
the ratio of the fatty acid component to water being in a range of 0.1:100 to 20:100 with respect to weight,
the composition having a pH in the range of 6 to 8 and
having a viscosity of 2,000 to 20,000 mPa*s at 23°C.

### Embodiment 3

The fatty acid component as described in the present text for use in a method of treating caries or reducing the risk of getting caries by reducing the lactic acid release of lactic acid producing bacteria in an oral biofilm in the mouth of a living human or animal,
the fatty acid component being applied in combination with water and amino acid(s),
the ratio of the fatty acid component to water being in a range of 0.1:100 to 10:100 with respect to weight,
the amino acid(s) being selected from glycine, leucine, isoleucine, lysine, methionine, phenylalanine, serine, threonine, valine, tryptophan and mixtures thereof,
the method of treating caries or reducing the risk of getting caries by reducing the lactic acid release comprising the step of applying the composition being applied to the oral biofilm for at least 1 min.

### Embodiment 4

The fatty acid as described in the present text for use in a method of treating caries or reducing the risk of getting caries by reducing the lactic acid release of lactic acid producing bacteria in an oral biofilm in the mouth of a living human or animal,
the fatty acid component being applied in combination with water,
the ratio of the fatty acid component to water being in a range of 0.1:100 to 10:100 with respect to weight,
the composition having a pH in the range of 6 to 8,
the method of treating caries or reducing the risk of getting caries by reducing the lactic acid release comprising the steps of applying fatty acid to the oral biofilm and/or hard dental tissue according to either of the following application schemes:
at least 2 times for at least 1 min within 24 hours,
for at least 1 hour within 24 hours, or
for at least 2 days.

### Embodiment 5

The fatty acid component as described in the present text for use in a method of treating caries or reducing the risk of getting caries by reducing the lactic acid release of lactic acid producing bacteria in an oral biofilm in the mouth of a living human or animal,
the fatty acid component being applied in combination with water and amino acid(s),
the ratio of the fatty acid component to water being in a range of 0.1:100 to 10:100 with respect to weight,
the amino acid being selected from
   glycine in an amount of 0.1 to 10 wt.%; or
   leucine in an amount of 0.1 to 5 wt.%; or
   isoleucine in an amount of 0.1 to 5 wt.%; or
   lysine in an amount of 0.1 to 10 wt.%; or
   methionine in an amount of 0.1 to 10 wt.%; or
   phenylalanine in an amount of 0.1 to 5 wt.%; or
   serine in an amount of 0.1 to 10 wt.%; or
   threonine in an amount of 0.1 to10 wt.%; or
   valine in an amount of 0.1 to 8 wt.%; or
   tryptophan in an amount of 0.1 to 2 wt.%; or
   mixtures thereof,
   wt.% with respect to the weight of the whole composition,
   the composition having a pH in the range of 6 to 8,
   the method of treating caries or reducing the risk of getting caries by reducing the lactic acid release comprising the steps of applying the fatty acid component to the oral biofilm with the aid of an application device selected from a dental tray, mouth guard or clear tray aligner.

### Embodiment 6

The fatty acid component as described in the present text for use in a method of treating caries or reducing the risk of getting caries by reducing the lactic acid release of lactic acid producing bacteria in an oral biofilm in the mouth of a living human or animal,
the fatty acid component being applied in combination with film-forming, gel-forming or paste-forming component(s),
the fatty acid component being applied to the oral biofilm and/or hard dental tissue for at least 2 days.

### Embodiment 7

The fatty acid component as described in the present text for use in a method of treating caries or reducing the risk of getting caries by reducing the lactic acid release of lactic acid producing bacteria in an oral biofilm in the mouth of a human being or animal,
the fatty acid component being selected from Butyric acid, 2-Ketobutyric acid, 2-Hydroxybutyric acid, Sodium 3-methyl-2-oxobutyrat, 2-Hydroxy-3-methylbutyric acid, 3-Methylbutryic acid, Pentanoic acid, 4-Oxopentanoic acid, 4-Methyl-2oxopentanoic acid, Hexanoic acid, 6-Hydroxyhexanoic acid, Heptanoic acid, Octanoic acid, 2-Hydroxy-octanoic acid, the respective salts, hydrates and mixtures thereof,
and being used in combination with water in a ratio of 0.1 to 10% with respect to weight,
the method of treating caries or reducing the risk of getting caries comprising the step of bringing the fatty acid component into contact with the oral biofilm according to either of the following application schemes:
at least 2 times for at least 1 min within 24 hours, or
for at least 1 hour within 24 hours, or
for at least for 2 days.

All these compositions can be used in a method or therapy described in the present text.

The complete disclosures of the patents, patent documents, and publications cited herein are incorporated by reference in their entirety as if each were individually incorporated. Various modifications and alterations to this invention will become apparent to those skilled in the art without departing from the scope and spirit of this invention. The above specification, examples and data provide a description of the manufacture and use of the compositions and methods of the invention. The invention is not limited to the embodiments disclosed herein. One skilled in the art will appreciate that many alternative embodiments of the invention can be made without departing from the spirit and scope of thereof.

The following examples are given to illustrate, but not limit, the scope of this invention.

### Examples

Unless otherwise indicated, all parts and percentages are on a weight basis, all water is deionized water (DEI-water), and all molecular weights are weight average molecular weight. Moreover, unless otherwise indicated all experiments were conducted at ambient conditions (23°C; 1013 mbar).

### Materials

**Table 1**

| Component | | Availability |
|---|---|---|
| | Short Fatty Acid Components | Sigma Aldrich |
| | C4 - fatty acids | |
| FA4-1 | 2-Ketobutyric acid | |
| FA4-2 | 2-Hydroxybutyric acid | |
| FA4-3 | Sodium 3-Methyl-2-oxobutyrat | |
| FA4-4 | 2-Hydroxy-3-methylbutyric acid | |
| FA4-5 | 3-Methylbutanic acid | |
| | C5 - fatty acids | |
| FA5-1 | Pentanoic acid | |
| FA5-2 | 4-Oxopentanoic acid | |
| FA5-3 | 4-Methyl-2oxopentanoic acid | |
| | C6 - Fatty acids | |
| FA6-1 | 6-Hydroxyhexanoic acid | |
| | C7 - Fatty acids | |
| FA7-1 | Heptanoic acid | |
| | C8 - Fatty acids | |
| FA8-1 | 2-Hydroxy-octanoic acid | |
| | C12 - Fatty acids | |
| FA12 | C12 - Dodecanoic acid | |
| Sucrose | Sugar | Sigma Aldrich |
| PBS | Phosphate Buffered Saline (Dulbecco 'A') | |
| | - Sodium Chloride (8g/IL) | |
| | - Potassium Chloride (0.2g/L) | Sigma Aldrich |
| | - Di-Sodiumhydrogenphosphate (1.15g/L) | Carl Roth |
| | - Potassiumdihydrogenphosphate (0.2g/L) | Honeywell |
| | - pH 7.3 +/- 0.2 | Acros Organics |
| | *Dulbecco and Vogt (1954), J. Exp. Med 99, 167-182 | |
| MCM | Mucine containing medium containing 1 % sucrose | |
| | - Lab-Lemco powder (1g/L) | Oxoid |
| | - Yeast extract (2g/L) | Oxoid |
| | - Proteose peptone (5g/L) | Oxoid |
| | - Hog gastic mucine Type III (2.5g/L) | Sigma Aldrich |
| | - Sodium Chloride (0.35g/L) | Sigma Aldrich |
| | - Calcium Chloride (0.2g/L) | VWR Chemicals |
| | - Potassium Chloride (0.2g/L) | Carl Roth |
| | - 40% Urea solution filter sterilized (1.25m/L) | |
| | ∘ Urea | Sigma Aldrich |
| | ∘ DEI-water | |
| | - Sucrose (10g/L) | Fisher Scientific |
| | - pH 6.9 +/-0,1 | |
| | *Pratten, Smith, Wilson (1998), J. Antimic. Chemoth: 42, 453-459 | |
| | *Badawi, Wilson, Pratten (2003), Biomaterials 24; 3345-3350 | |
| | Enzyme assay | |
| Glycylglycine | Glycylglycine (200mM) in di-water, pH 9.0 +/- 0,1 | |
| Buffer | - Glycylglycine (26.424g/L) | Carl Roth |
| | - DEI-water | |
| Enzyme solution | LDH containing solution with 2.5 U (+/-0,3) | |
| | - DEI-water (386g/kg) | |
| | - Glycerin (549g/kg) | Sigma Aldrich |
| | - Methyl 4-hydroxybenzoate sodium salt (0.59g/kg) | Acros Organics |
| | | Sigma Aldrich |
| | - Propyl-4-hydroxybenzoate sodium salt (0.17g/kg) | Carl Roth |
| | - Glycylglycine (44.57g/kg) | Sigma Aldrich |
| | - 40% Sodium hydroxide solution (0.04g/kg) | Sigma Aldrich |
| | - Pyruvate (0,0047g/kg) | Sigma Aldrich |
| | - Bovine serum albumine (0.1 g/kg) | Sigma Aldrich |
| | - Lactate dehydrogenase (2.5U) | |
| PMS/MTT | PMS/MTT containing solution, pH 2.5 (+/-0.1) | |
| | - Potassium chloride (1.49g/kg) | Carl Roth |
| | - 37% Hydrochloric acid (2g/kg) | Fisher Scientific |
| | - Tetrazoliumbromide (0.96g/kg) | Acros Organics |
| | - Phenazine Methosulfate (0.12g/kg) | Acros Organics |
| | - DEI-water | |
| NAD | NAD containing solution (31.5mM) | AppliChem |
| | - Nicotinamide adenine dinucleotide (21.5g/mL) | |
| | - DEI-water | |
| Human saliva | Daily fresh | |

### Methods

### Determination of Lactic Acid Release (LAR)

To determine the metabolic activity of the biofilm sample, the accumulation of lactic acid in the supernatant (1.0 ml PBS-solution with 5% sucrose per each biofilm sample disc) was measured by using the Lactic acid dehydrogenase (LDH)- Nicotinamide-adenine-dinucleotide (NAD)-Phenazine Methosulphate (PMS) - Thiazolyl blue tetrazolium bromide (MTT) - enzyme assay.

To ensure that only freshly produced lactic acid is determined, the biofilm samples were washed four times with 1.0 ml PBS solution for 30 sec each.

After 30 min the accumulated amount of lactic acid in the supernatant was measured by analyzing 50 µl of the supernatant.

The lactic acid was quantified by the lactic acid specific enzyme assay described above. Known correlation between measured enzyme activity and amount of lactic acid was used to translate measured enzyme activity into the amount of lactic acid present in sample of the supernatant.

In accordance to the validation of the in vitro human saliva microcosm biofilm model, a compound should lower the lactic acid release in permanent application of at least 40 or at least 45 or at least 50 % in comparison to the untreated biofilm control.

Negative number indicates that the release of lactic acid was reduced. A positive number indicates that the release of lactic acid was increased.

### Permanent Exposure Procedure

Bovine sample disks of about 10 x 10 mm (+/- 2mm) were incubated in a 24 well plate containing 1.8 ml medium in an incubator at 37°C and 60 rpm circular movement. The treatment composition described below were added by pipetting and removed by a suction pump.

### General Procedure:

- Day 1: Four-hours inoculation of bovine sample disks with saliva-mcm media (MCM = mucine containing medium plus 1 % sucrose) at 37 °C (without active substance).
- Fresh medium (MCM-1% sucrose) containing the respective amounts of fatty acid component as given in Table 2. This Treatment Composition was used for another 3-hour incubation.
- Fresh medium (MCM-1% sucrose and Treatment Composition) for storage for about 14h at 37 °C.
- Day 2: Fresh medium (MCM-1% sucrose and Treatment Composition) for 2-hour incubation.
- Fresh medium (MCM-1% sucrose and Treatment Composition) for another 2-hour incubation.
- Samples were taken after about 26 hours biofilm growth followed by lactic acid release (LAR).

For a permanent exposure procedure, the reduction in the lactic acid release should be at least 40 or at least 50 or at least 60%.

### Preparation of Treatment Compositions

Different Treatment Compositions (TCx) to be used for the Permanent Exposure Procedure were prepared by dissolving the respective fatty acid components in a liquid carrier medium (MCM) in the amounts given below in Table 1.

The Treatment Compositions (TCx) had the following compositions:

**Table 1:**

| Sample (TCx) | Fatty acid component [wt.%] | Mw [g/mol] |
|---|---|---|
| TC0 | 0 | n.a. |
| TC-FA4-1 | 5 | 102.9 |
| TC-FA4-2 | 5 | 126.09 |
| TC-FA4-3 | 5 | 138.10 |
| TC-FA4-4 | 1 | 118.13 |
| TC-FA4-5 | 5 | 102.13 |
| TC-FA5-1 | 2 | 102.13 |
| TC-FA5-2 | 5 | 116.12 |
| TC-FA5-3 | 3 | 130.14 |
| TC-FA6-1 | 1 | 132.16 |
| TC-FA7-1 | 0.25 | 130.18 |
| TC-FA8-1 | 1 | 160.21 |
| TC-FA12 | 0.003 | 200.32 |

The results for the Permanent Exposure Procedure are given in Table 2.

**Table 2**

| Sample (TCx) | Fatty acid component [wt.%] | LAR [%] |
|---|---|---|
| TC0 | 0 | 0 |
| TC-FA4-1 | 5 | -97.7 |
| TC-FA4-2 | 5 | -53.3 |
| TC-FA4-3 | 5 | -99.4 |
| TC-FA4-4 | 1 | -46.5 |
| TC-FA4-5 | 5 | -96.8 |
| TC-FA5-1 | 2 | -93.5 |
| TC-FA5-2 | 5 | -95.5 |
| TC-FA5-3 | 3 | -98.3 |
| TC-FA6-1 | 1 | -61.7 |
| TC-FA7-1 | 0.25 | -93.4 |
| TC-FA8-11 | 1 | -92.4 |
| TC-FA12 | 0.003 | +0.3 |

### Evaluation:

The short fatty acid components described in the present text are effective to lower the lactic acid release from in vitro human saliva microcosm biofilm.

## Claims

1. An oral care composition for treating caries or reducing the risk of getting caries,
the oral care composition comprising a fatty acid component or salts thereof contained in a solvent and/or carrier,
the fatty acid component comprising a carboxyl moiety which is attached to a C₃ to C₇ moiety
to which optionally one or more residues selected from methyl, methoxy or hydroxy can be attached, and/or
wherein one alky moiety of the C₃ to C₇ moiety is replaced by a keto moiety.

2. The oral care composition according to any of preceding claims, the fatty acid component being **characterized by** the following formula, the respective salts and hydrates thereof:
H₂CR⁷-(CHR⁶)ᵣ-(CHR⁵)_{q}-(CHR⁴)ₚ-(CHR³)ₒ-(CHR²)ₙ-(CHR¹)ₘ-COOH
with
m+n+o+p+q+r being independently selected from 0 or 1,
m+n+o+p+q+r ≥ 2,
R¹⁻⁶ being independently selected from H, CH₃, OH and OCH₃,
R⁷ being independently selected from H, OH,
wherein one of CHR^{x} can be replaced by a keto moiety,
wherein not more than two of CHR^{x} can be replaced by a CH₃, OH or OCH₃ moiety,
with x being 1 to 6.

3. The oral care composition according to any of preceding claims, the fatty acid component being selected from Butyric acid, 2-Ketobutyric acid, 2-Hydroxybutyric acid, 3-Hydroxybutyric acid, Sodium 3-methyl-2-oxobutyrat, 2-Hydroxy-3-methylbutyric acid, 3-Methylbutryic acid, Pentanoic acid, 4-Oxopentanoic acid, 4-Methyl-2oxopentanoic acid, Hexanoic acid, 6-Hydroxyhexanoic acid, Heptanoic acid, Octanoic acid, 2-Hydroxy-octanoic acid, the respective salts, hydrates and mixtures thereof.

4. The oral care composition according to any of the preceding claims, the fatty acid component being contained in a solvent, the solvent comprising water, the fatty acid component being present preferably in an amount of at least 0.1 wt.% with respect to the whole composition.

5. The oral care composition according to any of the preceding claims, the fatty acid component being contained in a carrier component not being water, the carrier component preferably comprising gel-forming agent(s), paste-forming agent(s) and film-forming agent(s).

6. The oral care composition according to any of the preceding claims comprising in addition at least one amino acid selected from glycine, leucine, isoleucine, lysine, methionine, phenylalanine, serine, threonine, valine, tryptophan and mixtures thereof.

7. Use of the fatty acid component as described in any of the preceding claims for preparing an oral care composition.

8. A kit of parts comprising
Part A comprising the fatty acid component as described in any of the preceding claims,
Part B comprising water and/or a carrier component,
Part C comprising optionally an application device.

9. The fatty acid component described in any of the preceding claims for use in a method of treating caries or reducing the risk of getting caries of a living human being or animal.

10. The fatty acid component for use as described in the preceding claim,
the method of treating caries or reducing caries being effected by reducing the lactic acid release of lactic acid releasing bacteria in an oral biofilm located in the mouth of a living human being or animal.

11. The fatty acid component for use in a method according to any of claims 9 to 10,
the method of treating caries or reducing the risk of getting caries being achieved by a process comprising the step of bringing the fatty acid component into contact with the oral biofilm, the oral biofilm being preferably located in marginal regions of hard dental tissue.

12. The fatty acid component for use in a method according to claim 11, the step of bringing the fatty acid component into contact with the oral biofilm being done by a step comprising one of the following treatment schemes:
for 1 to 5 min, or
for 1 to 5 min 2-times within 24 hours, or
for at least 1 hour within 24 hours.

13. The fatty acid component for use in a method according to any of claims 11 to 12, the step of bringing the fatty acid component into contact with the oral biofilm being done by rinsing, spraying, brushing, swabbing, coating or combinations thereof.

14. The fatty acid component for use in a method according to any of claims 11 to 13, the step of bringing the fatty acid component into contact with the oral biofilm being done with the aid of an application device, preferably with the aid of a dental tray, mouth guard or clear tray aligner.

15. The fatty acid component for use in a method according to any of claims 10 to 14, the fatty acid component being applied for a time period effective to reduce the lactic acid release of lactic acid producing bacteria in the oral biofilm by at least 40%.
